(19) **Europäisches Patentamt**
**European Patent Office**
**Office européen des brevets**

(11) **EP 2 770 051 B1**

(12) **EUROPEAN PATENT SPECIFICATION**

(45) Date of publication and mention
of the grant of the patent:
**27.09.2017 Bulletin 2017/39**

(21) Application number: **12842151.8**

(22) Date of filing: **22.10.2012**

(51) Int Cl.:
*C12N 5/10* (2006.01)    *C12N 5/00* (2006.01)
*C12N 5/071* (2010.01)    *C12N 5/0735* (2010.01)
*C12M 3/06* (2006.01)    *C12N 5/074* (2010.01)

(86) International application number:
**PCT/JP2012/077273**

(87) International publication number:
**WO 2013/058403 (25.04.2013 Gazette 2013/17)**

(54) **METHOD FOR CULTURING PLURIPOTENCY-MAINTAINED SINGLY DISPERSED CELLS BY MEANS OF LAMINAR FLOW**

VERFAHREN ZUR KULTIVIERUNG VON EINZEL DISPERGIERTEN ZELLEN MIT BEWAHRTER PLURIPOTENZ DURCH LAMINARE STRÖMUNG

MÉTHODE DE CULTURE DE CELLULES INDIVIDUELLEMENT DISPERSÉES ET MAINTENUES PLURIPOTENTES AU MOYEN D'UN FLUX LAMINAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.10.2011 JP 2011232097**

(43) Date of publication of application:
**27.08.2014 Bulletin 2014/35**

(73) Proprietor: **ARKRAY, Inc.**
**Minami-ku**
**Kyoto-shi**
**Kyoto 601-8045 (JP)**

(72) Inventors:
• **KOTERA, Hidetoshi**
**Kyoto-shi**
**Kyoto 606-8501 (JP)**
• **TATSUMI, Kazuya**
**Kyoto-shi**
**Kyoto 606-8501 (JP)**
• **YOKOKAWA, Ryuji**
**Kyoto-shi**
**Kyoto 606-8501 (JP)**
• **TADA, Takashi**
**Kyoto-shi**
**Kyoto 606-8501 (JP)**
• **NAGATA, Shogo**
**Kyoto-shi**
**Kyoto 606-8501 (JP)**

• **OKITSU, Teru**
**Kyoto-shi**
**Kyoto 606-8501 (JP)**
• **OKONOGI, Atsuhito**
**Kyoto-shi**
**Kyoto 602-0008 (JP)**
• **NODA, Yuichiro**
**Kyoto-shi**
**Kyoto 602-0008 (JP)**
• **NAKANISHI, Naoyuki**
**Kyoto-shi**
**Kyoto 602-0008 (JP)**
• **MATSUMURA, Taku**
**Kyoto-shi**
**Kyoto 602-0008 (JP)**
• **OSUMI, Takashi**
**Kyoto-shi**
**Kyoto 602-0008 (JP)**

(74) Representative: **Jones, Elizabeth Louise**
**Dehns**
**St Bride's House**
**10 Salisbury Square**
**London**
**EC4Y 8JD (GB)**

(56) References cited:
**JP-A- 2010 532 173**    **JP-A- 2011 147 387**
**US-A1- 2008 032 380**    **US-A1- 2010 112 691**

   **(Cont. next page)**

- **LUIS GERARDO VILLA-DIAZ ET AL: "Microfluidic culture of single human embryonic stem cell colonies", LAB ON A CHIP, vol. 9, no. 12, 1 January 2009 (2009-01-01), page 1749, XP055167964, ISSN: 1473-0197, DOI: 10.1039/b820380f**
- **G. MURUGAPPAN ET AL: "Human hematopoietic progenitor cells grow faster under rotational laminar flows", BIOTECHNOLOGY PROGRESS, vol. 26, no. 5, 22 April 2010 (2010-04-22), pages 1465-1473, XP055167950, ISSN: 8756-7938, DOI: 10.1002/btpr.440**
- **YI-CHIN TOH ET AL.: 'Fluid shear stress primes mouse embryonic stem cells for differentiation in a self-renewing environment via heparan sulfate proteoglycans transduction.' FASEB J. vol. 25, no. 4, April 2011, pages 1208 - 1217, XP055150429**
- **MASATOSHI INOUE ET AL.: 'Micro Kukan ga Saibo no Zoshokusei ni Ataeru Eikyo' JSME ANNUAL MEETING 10 September 2011, XP008173491**
- **MASATOSHI INOUE ET AL.: 'Micro Kukan deno Saibo Baiyo ni Okeru Butsuriteki/Kagakuteki Eikyo no Teiryo Hyoka' PROCEEDINGS OF THE 28TH SENSOR SYMPOSIUM ON SENSORS, MICROMACHINES, AND APPLIED SYSTEMS 26 September 2011, pages 85 - 88, XP008173480**

SEQUENCE LISTING

<110> ARKRAY, INC.

<120> METHOD FOR CULTURING PLURIPOTENCY-MAINTAINED SINGLY DISPERSED CELLS BY MEANS OF LAMINAR FLOW

<130> 42.34.120161

<140> EP12842151.8
<141> 2012-10-22

<150> JP2011-232097
<151> 2011-10-21

<160> 16

<170> PatentIn version 3.3

<210> 1
<211> 26
<212> DNA
<213> Artificial sequence

<220>
<223> primer

<400> 1
gcactgtact cctcggtccc tttccc          26

<210> 2
<211> 26
<212> DNA
<213> Artificial sequence

<220>
<223> primer

<400> 2
cttccctcca accagttgcc ccaaac          26

<210> 3
<211> 26
<212> DNA
<213> Artificial sequence

<220>
<223> primer

<400> 3
gggaaatggg aggggtgcaa aagagg         26

<210> 4
<211> 26
<212> DNA
<213> Artificial sequence

<220>
<223> primer

<400> 4
ttgcgtgagt gtggatggga ttggtg         26

<210> 5
<211> 25
<212> DNA
<213> Artificial sequence

<220>
<223> primer

<400> 5
cagccctgat tcttccacca gtccc          25

<210> 6
<211> 25
<212> DNA
<213> Artificial sequence

<220>
<223> primer

<400> 6
tggaaggttc ccagtcgggt tcacc          25

<210> 7
<211> 23
<212> DNA
<213> Artificial sequence

<220>
<223> primer

<400> 7
cttatgctac gtaaaggagc ggg            23

<210> 8
<211> 24
<212> DNA

<213> Artificial sequence

<220>
<223> primer

<400> 8
gtgccaaccc aggtcccgga agtt          24

<210> 9
<211> 24
<212> DNA
<213> Artificial sequence

<220>
<223> primer

<400> 9
cagatcctaa acagctcgca gatt          24

<210> 10
<211> 23
<212> DNA
<213> Artificial sequence

<220>
<223> primer

<400> 10
gcgtacgcaa attaaagtcc aga          23

<210> 11
<211> 20
<212> DNA
<213> Artificial sequence

<220>
<223> primer

<400> 11
gtggggaact cagcaaatac          20

<210> 12
<211> 20
<212> DNA
<213> Artificial sequence

<220>
<223> primer

<400> 12
gcatcatatc atccatgctg          20

<210> 13
<211> 20
<212> DNA
<213> Artificial sequence

<220>

<223> primer

<400> 13
gcctagtgtt gtgtcaagac        20

<210> 14
<211> 23
<212> DNA
<213> Artificial sequence

<220>
<223> primer

<400> 14
ggtgcaagaa taagatttat ggc        23

<210> 15
<211> 23
<212> DNA
<213> Artificial sequence

<220>
<223> primer

<400> 15
cttcttttgc gtcgccagcc gag        23

<210> 16
<211> 22
<212> DNA
<213> Artificial sequence

<220>
<223> primer

<400> 16
cagccttgac ggtgccatgg aa        22

**Description**

TECHNICAL FIELD

The one aspect of the present invention relates to a method for culturing dispersed single pluripotent cells. More specifically, the one aspect of the present invention relates to a method for culturing dispersed single human embryonic stem cells (hESCs) or human induced pluripotent stem cells (hiPSCs).

BACKGROUND ART

hESCs (Non-patent Document 1) and hiPSCs (Non-patent Document 2) are cells whose use in regenerative medicine in the near future is expected. In cases where these cells are dispersed into single cells by destruction of cell-cell adhesion under normal maintenance culture conditions, myosin hyperactivation occurs, and this leads to formation of blebs on the cells, followed by the occurrence of cell death through apoptosis. This caspase-dependent cell death is suppressed by suppression of Rho and the activity of Rac by signaling from E-cadherin-mediated cell-cell adhesion (Non-patent Document 3). Such extracellular signaling via cell-cell interaction plays an important role in culture of dispersed single hiPSCs or hESCs. Therefore, in cloning of hESC and hiPSC, a ROCK inhibitor (Y-27632) and a myosin inhibitor (Blebbistatin) are used as antiapoptotic agents for performing culture of dispersed single cells.

In recent years, it has been reported that the liquid flow generated by cilia in a vertebrate embryo is involved in asymmetric differentiation of the embryo (Non-patent Document 4), and it has been revealed that response to external stimuli on cells is involved in determination of the fate of the cells. Further, it has also been reported that shear stress via stimulation of heparin sulfate proteoglycan on the cell surface is involved in self-renewal of mouse ES cells (Non-patent Document 5). However, unlike mouse ES cells, hESCs and hiPSCs are called epiblast stem cells, and these cells have therefore been reported to have different properties such as different reactions in response to cytokines (Non-patent Document 6). Therefore, at present, response of hESCs and hiPSCs to shear stress cannot be predicted. Thus, a method for cloning of dispersed single hESCs and hiPSCs by culture of the dispersed single cells utilizing shear stress is unknown at present.

PRIOR ART DOCUMENTS

[Non-patent Documents]

Non-patent Document 1: Thomson, J. A. et al. Science, 1998, 282:1145-1147
Non-patent Document 2: Takahashi K and Yamanaka S. Cell. 2006, 126:663-676
Non-patent Document 3: Ohgushi M, st al. Cell Stem Cell. 2010, 7:225-239
Non-patent Document 4: Burdine RD and Schier AF. Genes Dev. 2000, 14:763-776
Non-patent Document 5: Toh YC and Voldman J. FASEB J. 201, 25:1208-1217
Non-patent Document 6: Tesar PJ. st al. Nature. 2007, 448:196-199

SUMMARY OF THE INVENTION

PROBLEMS TO BE SOLVED BY THE INVENTION

The one aspect of the present invention aims to provide a novel cloning method for hESCs and hiPSCs, which method is based on culture of dispersed single cells without addition of an anti-apoptotic agent. More specifically, the one aspect of the present invention aims to provide a cloning method for hESCs and hiPSCs based on culture of dispersed single cells utilizing shear stress.

MEANS FOR SOLVING THE PROBLEMS

In order to solve the above problem, the present inventors intensively studied to discover for the first time that cloning of hESCs and hiPSCs by culture of dispersed single cells can be achieved by employing laminar flow conditions in the culture of hESCs and hiPSCs. The present invention was completed based on such knowledge.

That is, the present invention provides:

(1) a method for culturing dispersed single pluripotent cells wherein said dispersed cells are separated cells that do not show joining of two or more cells via cadherin, said method comprising culturing adhered dispersed cells under laminar flow conditions without addition of an anti-apoptotic agent;

(2) the method according to (1) wherein the cells are cultured on Matrigel;

(3) the method according to (1) or (2), wherein an embryonic stem cell-maintenance medium is allowed to flow as a laminar flow;

(4) the method according to (3), wherein the embryonic stem cell-maintenance medium comprises a culture supernatant of fibroblasts;

(5) the method according to any one of (1) to (4), wherein the cells are cultured in a cylindrical container whose cross-section is a circle having a diameter of not more than 1 mm;

(6) the method according to (5), wherein the cells are cultured in a container having a width of 0.1 mm to 1 mm, height of 0.1 mm to 1 mm and length of 5 to 40 mm;

(7) the method according to (6), wherein the cells are cultured in a container having a width of 0.5 mm, height of 0.5 mm and length of 20 mm;

(8) the method according to any one of (1) to (7), wherein the laminar flow has a flow rate of not more than 50 $\mu$l/min.;

(9) the method according to (8), wherein the laminar flow has a flow rate of 1 nl/min. to 50 $\mu$l/min.;

(10) the method according to (8), wherein the laminar flow has a flow rate of 50 nl/min. to 5 $\mu$l/min.;

(11) the method according to any one of (1) to (10), wherein the laminar flow has a flow rate that causes a microshear stress of not more than $8 \times 10^{-3}$ Pa on the cell surface;

(12) the method according to any one of (1) to (11), wherein the laminar flow has a flow rate that causes a microshear stress of not less than $8 \times 10^{-5}$ Pa on the cell surface;

(13) the method according to any one of (1) to (12), wherein the pluripotent cells are human pluripotent cells;

(14) the method according to (13), wherein the pluripotent cells are embryonic stem cells or induced pluripotent stem cells; and

(15) the method according to any one of claims (1) to (14), wherein the laminar flow is produced with a peristaltic pump.

EFFECT OF THE INVENTION

In the culture method of one aspect of the present invention, pluripotent cells are cultured in a state where the cells are dispersed as single cells, to enable production of their cloned cells. Therefore, one aspect of the present invention enables quality control of iPSCs for application to regenerative medicine, improvement of the efficiency of gene manipulation, improvement of the efficiency of cloning, and the like.

BRIEF DESCRIPTION OF THE DRAWINGS

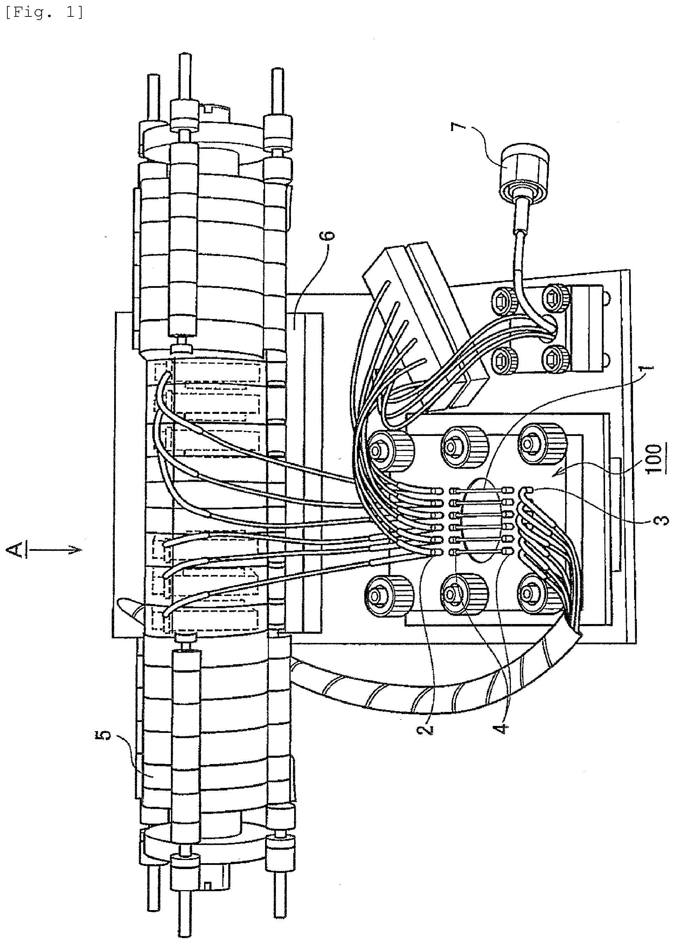

Fig. 1 is a diagram illustrating an embodiment of a microfluidic device.

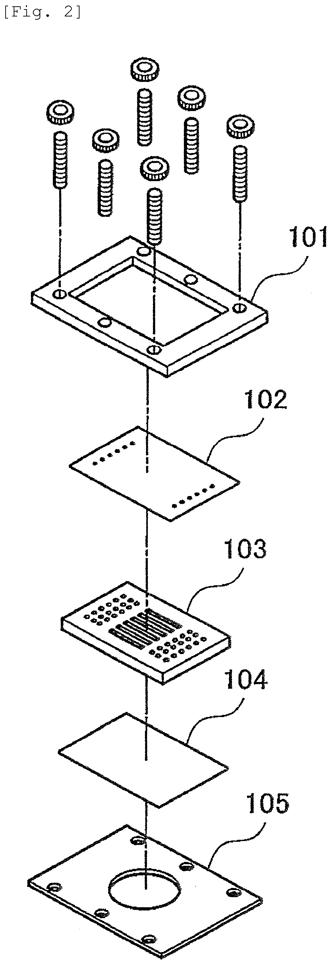

Fig. 2 is a diagram illustrating construction of a microfluidic chip.

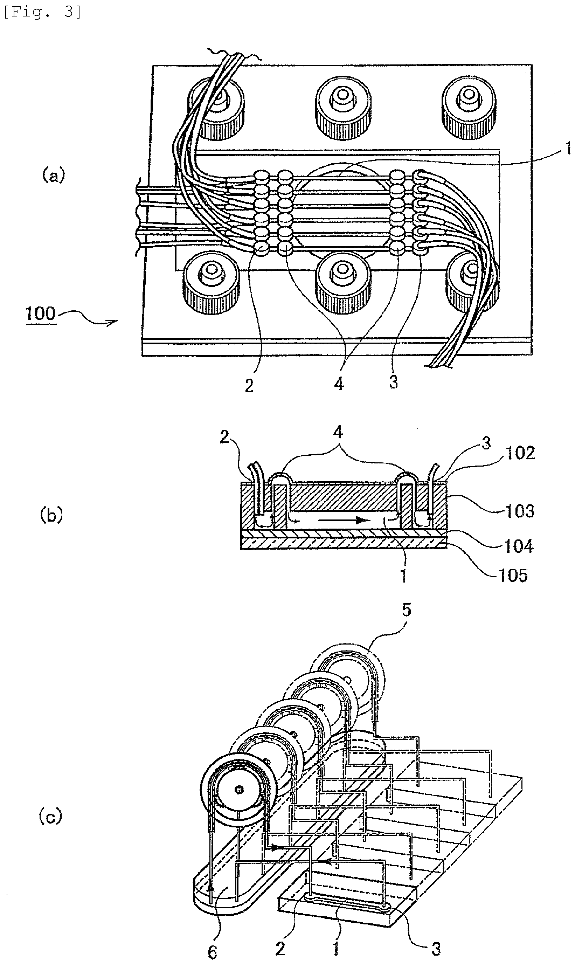

Fig. 3 illustrates an enlarged view of the microfluidic chip (a), and diagrams illustrating the flow of the fluid in the microfluidic chip (b and c). Each arrowhead indicates the direction of flow of the medium. b illustrates a cross-sectional view of the channel portion, and c illustrates the flow in which the medium is pumped up by a peristaltic pump 5 from a reservoir 6 into a channel 1, and the medium is then refluxed from the channel 1 into the reservoir 6.

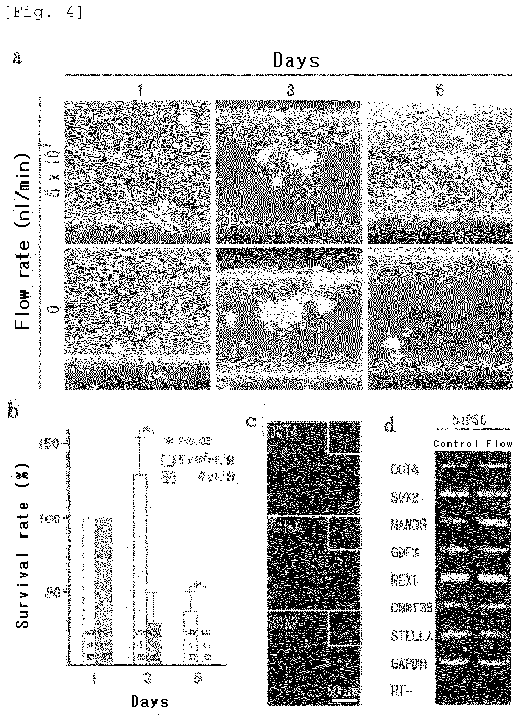

Fig. 4 is a diagram illustrating survival of human induced pluripotent stem cells (hiPSCs) in the microfluidic device. (a) illustrates the colony morphology of hiPSCs under culture conditions with and without a nanofluidic flow of the medium (photographs). (b) illustrates comparison between the survival rates of hiPSCs under nanofluidic flow conditions and static conditions. (c) illustrates expression of a pluripotency marker protein in hiPSCs that survived under nanofluidic flow conditions, as revealed by immunohistochemistry (photographs). (d) illustrates expression of pluripotency marker genes as revealed by RT-PCR (photographs).

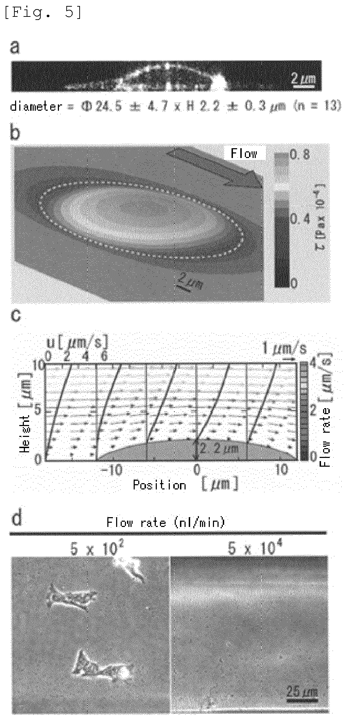

Fig. 5 illustrates evaluation of shear stress on hiPSCs (human induced pluripotent stem cells) under nanofluidic flow conditions. (a) illustrates a representative confocal section of hiPSCs cultured in a channel in the microfluidic device (photograph). $\varphi$: diameter, H: height. (b) illustrates simulation of the distribution of shear stress on hiPSCs adhering to the bottom of a channel. The cell boundary is emphasized with a white dotted line. The gray arrowhead indicates the direction of fluid flow at 500 nl/min. (c) illustrates simulation of the velocity vector and size around hiPSCs adhering to the bottom of a channel. (d) illustrates survival of hiPSCs in a nanofluidic flow of the medium rather than a microfluidic flow (pictures). The hiPSCs are detached from the culture bottom 24 hours after the plating, but can survive with normal cell morphology under the nanofluidic flow conditions.

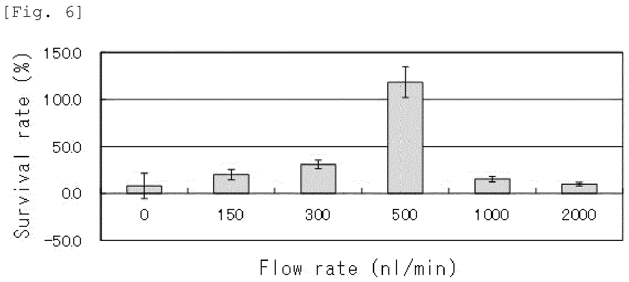

Fig. 6 illustrates the survival rates of hiPSCs at various flow rates. The survival rate is the ratio of the number of Nanog-positive living cells on Day 3 to the number of those cells on Day 1. Each survival rate is represented as the mean of n=4. Each error bar represents the standard deviation.

BEST MODE FOR CARRYING OUT THE INVENTION

<Pluripotent Cells>

The term "pluripotent cell" used in the present invention means a cell having a capacity to differentiate into a plurality of types of cells. Examples of the pluripotent cell include, but are not limited to, embryonic stem cells (ESCs), germline stem cells (GSCs), embryonic germ cells (EGCs), induced pluripotent stem cells (iPSCs), pluripotent cells derived from cultured fibroblasts and bone marrow stem cells (Muse cells), and somatic stem cells. The pluripotent cells may be derived from various organisms. They are preferably pluripotent cells derived from a mammal including human, more preferably mouse-derived pluripotent cells or primate-derived pluripotent cells. The pluripotent cells are particularly preferably derived from human.

(A) Embryonic Stem Cells

ES cells are stem cells that can be established from the inner cell mass of an early embryo (for example, blastocyst) of a mammal such as human or mouse, which cells have pluripotency and growth ability by self-renewal. ES cells can be embryo-derived stem cells originated from the inner cell mass of a blastocyst, which is the embryo formed following the 8-cell stage and the morula stage of a fertilized egg, and ES cells have the ability to differentiate into any cells constituting an adult, that is, the so called pluripotency of differentiation, and growth ability by self-renewal. ES cells were discovered in mice in 1981 (M. J. Evans and M. H. Kaufman (1981), Nature 292:154-156), and this was followed by establishment of ES cell lines of primates such as human and monkey (J. A. Thomson et al. (1998), Science 282:1145-1147; J. A. Thomson et al. (1995), Proc. Natl. Acad. Sci. USA, 92:7844-7848; J. A. Thomson et al. (1996), Biol. Reprod., 55:254-259; J. A. Thomson and V. S. Marshall (1998), Curr. Top. Dev. Biol., 38:133-165).
ES cells can be established by removing the inner cell mass from the blastocyst of a fertilized egg of a subject animal, followed by culturing the inner cell mass on feeder fibroblasts. The cells can be maintained by subculturing using a medium supplemented with a substance(s) such as leukemia inhibitory factor (LIF) and/or basic fibroblast growth factor (bFGF). Methods of establishment and maintenance of human and monkey ES cells are described in, for example, USP 5,843,780; Thomson JA, et al. (1995), Proc Natl. Acad. Sci. U S A. 92:7844-7848; Thomson JA, et al. (1998), Science. 282:1145-1147; H. Suemori et al. (2006), Biochem. Biophys. Res. Commun., 345:926-932; M. Ueno et al. (2006), Proc. Natl. Acad. Sci. USA, 103:9554-9559; H. Suemori et al. (2001), Dev. Dyn., 222:273-279; H. Kawasaki et al. (2002), Proc. Natl. Acad. Sci. USA, 99:1580-1585; and Klimanskaya I, et al. (2006), Nature. 444:481-485.
In terms of the medium for preparation of ES cells, human ES cells can be maintained, for example, using DMEM/F-12 medium supplemented with 0.1 mM 2-mercaptoethanol, 0.1 mM non-essential amino acids, 2 mM L-glutamic acid, 20% KSR and 4 ng/ml bFGF at 37°C under a moist atmosphere of 2% $CO_2$/98% air (O. Fumitaka et al. (2008), Nat. Biotechnol., 26:215-224). Further, ES cells need to be subcultured every 3 to 4 days, and the subculture can be carried out using 0.25% trypsin and 0.1 mg/ml collagenase IV in PBS supplemented with 1 mM $CaCl_2$ and 20% KSR.
Selection of ES cells can be generally carried out by the Real-Time PCR method using as an index/indices expression of a gene marker(s) such as alkaline phosphatase, Oct-3/4 and/or Nanog. In particular, for selection of human ES cells, expression of a gene marker(s) such as OCT-3/4, NANOG and/or ECAD can be used as an index/indices (E. Kroon et al. (2008), Nat. Biotechnol., 26:443-452).
In terms of human ES cell lines, for example, WA01(H1) and WA09(H9) can be obtained from WiCell Research Institute, and KhES-1, KhES-2 and KhES-3 can be obtained from Institute for Frontier Medical Sciences, Kyoto University (Kyoto, Japan).

(B) Germline Stem Cells

Germline stem cells are pluripotent stem cells derived from testis, and play a role as the origin for spermatogenesis. Similarly to ES cells, these cells can be induced to differentiate into various series of cells, and, for example, have a property to enable preparation of a chimeric mouse by transplantation of the cells to a mouse blastocyst (M. Kanatsu-Shinohara et al. (2003) Biol. Reprod., 69:612-616; K. Shinohara et al. (2004), Cell, 119:1001-1012). Germline stem cells are capable of self-renewal in a medium containing glial cell line-derived neurotrophic factor (GDNF), and, by repeating subculture under the same culture conditions as those for ES cells, germline stem cells can be obtained (Masanori Takehashi et al. (2008), Experimental Medicine, 26(5) (extra edition):41-46, Yodosha (Tokyo, Japan)).

(C) Embryonic Germ Cells

Embryonic germ cells are established from fetal primordial germ cells and have pluripotency similarly to ES cells. They can be established by culturing primordial germ cells in the presence of substances such as LIF, bFGF and stem cell factor (Y. Matsui et al. (1992), Cell, 70:841-847; J. L. Resnick et al. (1992), Nature, 359:550-551).

(D) Induced Pluripotent Stem Cells

Induced pluripotent stem (iPS) cells can be prepared by introducing specific reprogramming factors to somatic cells, which reprogramming factors are in the form of DNA or protein. iPS cells are somatic cell-derived artificial stem cells having properties almost equivalent to those of ES cells, such as pluripotency of differentiation and growth ability by self-renewal (K. Takahashi and S. Yamanaka (2006) Cell, 126:663-676; K. Takahashi et al. (2007), Cell, 131:861-872; J. Yu et al. (2007), Science, 318:1917-1920; Nakagawa, M. et al., Nat. Biotechnol. 26:101-106 (2008); WO 2007/069666). The reprogramming factors may be constituted by genes or gene products thereof, or non-coding RNAs, which are expressed specifically in ES cells; or genes or gene products thereof, non-coding RNAs or low molecular weight compounds, which play important roles in maintenance of the undifferentiated state of ES cells. Examples of the genes included in the reprogramming factors include Oct3/4, Sox2, Sox1, Sox3, Sox15, Sox17, Klf4, Klf2, c-Myc, N-Myc, L-Myc, Nanog, Lin28, Fbx15, ERas, ECAT15-2, Tel1, beta-catenin, Lin28b, Sall1, Sall4, Esrrb, Nr5a2, Tbx3 and Glis1, and these reprogramming factors may be used either alone or in combination. Examples of the combinations of the reprogramming factors include those described in WO2007/069666; WO2008/118820; WO2009/007852; WO2009/032194; WO2009/058413; WO2009/057831; WO2009/075119; WO2009/079007; WO2009/091659; WO2009/101084; WO2009/101407; WO2009/102983; WO2009/114949; WO2009/117439; WO2009/126250; WO2009/126251; WO2009/126655; WO2009/157593; WO2010/009015; WO2010/033906; WO2010/033920; WO2010/042800; WO2010/050626; WO2010/056831; WO2010/068955; WO2010/098419; WO2010/102267; WO 2010/111409; WO2010/111422; WO2010/115050; WO2010/124290; WO2010/147395; WO2010/147612; Huangfu D, et al. (2008), Nat. Biotechnol., 26: 795-797; Shi Y, et al. (2008), Cell Stem Cell, 2: 525-528; Eminli S, et al. (2008), Stem Cells. 26:2467-2474; Huangfu D, et al. (2008), Nat Biotechnol. 26:1269-1275; Shi Y, et al. (2008), Cell Stem Cell, 3, 568-574; Zhao Y, et al. (2008), Cell Stem Cell, 3:475-479; Marson A, (2008), Cell Stem Cell, 3, 132-135; Feng B, et al. (2009), Nat Cell Biol. 11:197-203; R.L. Judson et al., (2009), Nat. Biotech., 27:459-461; Lyssiotis CA, et al. (2009), Proc Natl Acad Sci U S A. 106:8912-8917; Kim JB, et al. (2009), Nature. 461:649-643; Ichida JK, et al. (2009), Cell Stem Cell. 5:491-503; Heng JC, et al. (2010), Cell Stem Cell. 6:167-74; Han J, et al. (2010), Nature. 463:1096-100; Mali P, et al. (2010), Stem Cells. 28:713-720; and Maekawa M, et al. (2011), Nature. 474:225-9.

Examples of the above-described reprogramming factors also include histone deacetylase (HDAC) inhibitors [for example, low molecular weight inhibitors such as valproic acid (VPA), trichostatin A, sodium butyrate, MC 1293 and M344; and nucleic acid-type expression inhibitors such as siRNAs and shRNAs against HDAC (e.g., HDAC1 siRNA Smartpool (Millipore) and HuSH 29mer shRNA Constructs against HDAC1 (OriGene)], MEK inhibitors (for example, PD184352, PD98059, U0126, SL327 and PD0325901), Glycogen synthase kinase-3 inhibitors (for example, Bio and CHIR99021), DNA methyltransferase inhibitors (for example, 5'-azacytidine), histone methyltransferase inhibitors (for example, low molecular weight inhibitors such as BIX-01294; and nucleic acid-type expression inhibitors such as siRNAs and shRNAs against Suv39hl, Suv39h2, SetDBl and G9a), L-channel calcium agonists (for example, Bayk8644), butyric acid, TGFβ inhibitors or ALK5 inhibitors (for example, LY364947, SB431542, 616453 and A-83-01), p53 inhibitors (for example, siRNAs and shRNAs against p53), ARID3A inhibitors (for example, siRNAs and shRNAs against ARID3A), miRNAs such as miR-291-3p, miR-294, miR-295 and mir-302, Wnt Signaling (for example, soluble Wnt3a), neuropeptide Y, prostaglandins (for example, prostaglandin E2 and prostaglandin J2), hTERT, SV40LT, UTF1, IRX6, GLIS1, PITX2 and DMRTB1, which are employed for enhancing the establishment efficiency, and, in the present description, these factors employed for the purpose of enhancement of the establishment efficiency are not particularly distinguished from reprogramming factors.

In cases where the reprogramming factors are in the form of protein, the reprogramming factors may be introduced into somatic cells by a method such as lipofection, fusion with a cell membrane-permeable peptide (e.g., HIV-derived TAT or polyarginine), or microinjection. In cases where the reprogramming factors are in the form of DNA, the reprogramming factors may be introduced into somatic cells by a method such as use of a vector including virus, plasmid and artificial chromosome vectors; lipofection; use of liposome; or microinjection. Examples of the virus vectors include retrovirus vectors, lentivirus vectors (these are described in Cell, 126, pp. 663-676, 2006; Cell, 131, pp. 861-872, 2007; and Science, 318, pp. 1917-1920, 2007), adenovirus vectors (Science, 322, 945-949, 2008), adeno-associated virus vectors and Sendai virus vectors (WO 2010/008054). Examples of the artificial chromosome vectors include human artificial chromosomes (HACs), yeast artificial chromosomes (YACs), and bacterial artificial chromosomes (BACs and PACs). Examples of the plasmids which may be used include plasmids for mammalian cells (Science, 322:949-953, 2008). The vectors may contain a regulatory sequence(s) such as a promoter, enhancer, ribosome binding sequence, terminator and/or polyadenylation site to enable expression of the nuclear reprogramming factors; and, as required, a sequence of a selection marker such as a drug resistance gene (e.g., kanamycin-resistant gene, ampicillin-resistant gene or puromycin-resistant gene), thymidine kinase gene or diphtheria toxin gene; a gene sequence of a reporter such as the green-fluorescent protein (GFP), β-glucuronidase (GUS) or FLAG; and/or the like. Further, in order to remove, after introduction of the above vector into somatic cells, the genes encoding the reprogramming factors, or both the promoters and the genes encoding the reprogramming factors linked thereto, the vector may have LoxP sequences upstream and downstream of these sequences.

Further, in cases where the reprogramming factors are in the form of RNA, each reprogramming factor may be introduced into somatic cells by a method such as lipofection or microinjection, and an RNA into which 5-methylcytidine and pseudouridine (TriLink Biotechnologies) are incorporated may be used in order to suppress degradation (Warren L, (2010) Cell Stem Cell. 7:618-630).

Examples of the medium for induction of the iPS cells include DMEM, DMEM/F12 and DME media supplemented with 10 to 15% FBS (these media may further contain LIF, penicillin/streptomycin, puromycin, L-glutamine, non-essential amino acids, β-mercaptoethanol and/or the like, as appropriate); and commercially available media [for example, a medium for culturing mouse ES cells (TX-WES medium, Thromb-X), medium for culturing primate ES cells (medium for primate ES/iPS cells, ReproCELL) and serum-free medium (mTeSR, Stemcell Technology)].

Examples of the culture method include a method wherein somatic cells and reprogramming factors are brought into contact with each other at 37°C in the presence of 5% $CO_2$ in DMEM or DMEM/F12 medium supplemented with 10% FBS, and the cells are cultured for about 4 to 7 days, followed by plating the cells on feeder cells (e.g., mitomycin C-treated STO cells or SNL cells) and starting culture in a bFGF-containing medium for culturing primate ES cells about 10 days after the contact between the somatic cells and the reprogramming factors, thereby allowing iPS-like colonies to appear about 30 to about 45 days after the contact, or later.

Alternatively, the cells may be cultured at 37°C in the presence of 5% $CO_2$ on feeder cells (e.g., mitomycin C-treated STO cells or SNL cells) in DMEM medium supplemented with 10% FBS (this medium may further contain LIF, penicillin/streptomycin, puromycin, L-glutamine, non-essential amino acids, β-mercaptoethanol and/or the like, as appropriate) for about 25 to about 30 days or longer, to allow ES-like colonies to appear. Preferred examples of the culture method include a method wherein the somatic cells themselves to be reprogrammed are used instead of the feeder cells (Takahashi K, et al. (2009), PLoS One. 4:e8067 or WO2010/137746), and a method wherein an extracellular matrix (e.g., Laminin-5 (WO2009/123349) or Matrigel (BD)) is used instead.

Other examples of the culture method include a method wherein culture is carried out using a serum-free medium (Sun N, et al. (2009), Proc Natl Acad Sci U S A. 106:15720-15725). Further, in order to enhance the establishment efficiency, iPS cells may be established under low oxygen conditions (at an oxygen concentration of 0.1% to 15%) (Yoshida Y, et al. (2009), Cell Stem Cell. 5:237-241 or WO2010/013845). During the culture, the medium is replaced with a fresh medium once every day from Day 2 of the culture. The number of somatic cells used for nuclear reprogramming is not restricted, and usually within the range of about $5 \times 10^3$ to about $5 \times 10^6$ cells per 100-cm² area on the culture dish.

iPS cells may be selected based on the shape of each formed colony. On the other hand, in cases where a drug resistance gene to be expressed in conjunction with a gene that is expressed when a somatic cell was reprogrammed (e.g., Oct3/4 or Nanog) was introduced as a marker gene, established iPS cells can be selected by culturing the cells in a medium containing the corresponding drug (selection medium). Further, iPS cells can be selected by observation under a fluorescence microscope in cases where the marker gene is the gene of a fluorescent protein; by adding a luminescent substrate in cases where the marker gene is the gene of luminous enzyme; or by adding a coloring substrate in cases where the marker gene is the gene of a coloring enzyme.

The term "somatic cells" used in the present description means any animal cells (preferably cells of mammals including human) excluding germ-line cells and totipotent cells such as eggs, oocytes and ES cells. Examples of the somatic cells include, but are not limited to, any of fetal somatic cells, neonatal somatic cells, and healthy or diseased mature somatic cells, as well as any of primary cultured cells, subcultured cells and established cell lines. Specific examples of the somatic cells include (1) tissue stem cells (somatic stem cells) such as neural stem cells, hematopoietic stem cells, mesenchymal stem cells and dental pulp stem cells; (2) tissue progenitor cells; and (3) differentiated cells such as lymphocytes, epithelial cells, endothelial cells, muscle cells, fibroblasts (skin cells and the like), hair cells, hepatic cells, gastric mucosal cells, enterocytes, spleen cells, pancreatic cells (pancreatic exocrine cells and the like), brain cells, lung cells, kidney cells and adipocytes.

In cases where iPS cells are used as the material for cells to be transplanted, somatic cells whose HLA genotype is the same or substantially the same as that of the individual to which the cells are to be transplanted are preferably used with a view to prevention of the rejection reaction. The term "substantially the same" herein means that the HLA genotype is matching to an extent at which the immune reaction against the transplanted cells can be suppressed with an immunosuppressive agent. For example, the somatic cells have matched HLA types at the 3 loci HLA-A, HLA-B and HLA-DR, or at the 4 loci further including HLA-C.

(F) Multilineage-differentiating Stress Enduring Cells (Muse Cells)

Muse cells are pluripotent stem cells produced by the method described in WO2011/007900. More specifically, Muse cells are cells having pluripotency obtained by subjecting fibroblasts or bone marrow stromal cells to trypsin treatment for a long period, preferably to trypsin treatment for 8 hours or 16 hours, followed by suspension culture of the treated cells. Muse cells are positive for SSEA-3 and CD105.

<Method for Culturing Dispersed Single Cells>

The term "method for culturing dispersed single cells" used in the present invention means a method for culturing and growing dispersed single cells, and includes an operation of cloning by growing separated cells that do not show joining of 2 or more cells via cadherin. For the dispersion of cells, a mechanical method may be used, or an agent may be used. Examples of the agent used in this process include an enzyme having protease activity or reagents containing EDTA. Specific examples of the agent include, but are not limited to, trypsin/EDTA, Accutase (TM) and Accumax (TM).

In one aspect of the present invention, the culture of dispersed single pluripotent cells is provided as a method that is carried out under laminar flow conditions. The laminar flow can be achieved by allowing a medium to flow on pluripotent cells adhered to a cell culture vessel. The cells grown by culture under such conditions preferably maintain pluripotency. In one aspect of the present invention, the maintenance of pluripotency can be confirmed by observing continuous expression of a pluripotency marker(s) in the cells. Examples of the pluripotency marker(s) include, but are not limited to, Oct3/4 and Nanog.

The "laminar flow" in the present invention means that the line of flow of the fluid is parallel to the wall surface, that is, it means a non-turbulent flow field. Preferably, the laminar flow is a flow field in which the flow rate decreases as the flow gets closer to the wall, and the flow rate is constant when the flow is away from the wall surface at more than a certain distance. Therefore, in the present invention, the flow rate of laminar flow means the constant flow rate observed when the flow is away from the wall surface at more than a certain distance.

For example, the flow rate of laminar flow is the flow rate measured at a point that is not less than 10 $\mu$m distant from the cell surface. Examples of the flow rate of laminar flow in one aspect of the present invention include, but are not limited to, not more than 50 $\mu$l/minute, not more than 40 $\mu$l/minute, not more than 30 $\mu$l/minute, not more than 25 $\mu$l/minute, not more than 20 $\mu$l/minute, not more than 10 $\mu$l/minute, not more than 5 $\mu$l/minute, not more than 3 $\mu$l/minute, not more than 2.5 $\mu$l/minute, not more than 2 $\mu$l/minute, not more than 1 $\mu$l/minute, not more than 900 nl/minute, not more than 800 nl/minute, not more than 700 nl/minute, not more than 650 nl/minute, not more than 600 nl/minute, not more than 500 nl/minute, not more than 400 nl/minute, not more than 300 nl/minute, not more than 200 nl/minute, not more than 100 nl/minute, not more than 50 nl/minute, not more than 25 nl/minute, and not more than 1 nl/minute. The flow rate is preferably not more than 5 $\mu$l/minute. The flow rate is more preferably not more than 3 $\mu$l/minute, or not more than 2.5 $\mu$l/minute, at a point not less than 10 $\mu$m distant from the cell surface. The flow rate is still more preferably not more than 2 $\mu$l/minute. Further, the flow rate is still more preferably not more than 1 $\mu$l/minute. Further, the flow rate is still more preferably not more than 650 nl/minute, or not more than 600 nl/minute, at a point not less than 10 $\mu$m distant from the cell surface. The flow rate is still more preferably 500 nl/minute or near 500 nl/minute, at a point not less than 10 $\mu$m distant from the cell surface. Further, examples of the flow rate of laminar flow include, but are not limited to, not less than 1 nl/minute, not less than 25 nl/minute, not less than 50 nl/minute, not less than 100 nl/minute, not less than 120 nl/minute, not less than 150 nl/minute, not less than 200 nl/minute, not less than 300 nl/minute, not less than 400 nl/minute, not less than 450 nl/minute, not less than 500 nl/minute, not less than 600 nl/minute, not less than 700 nl/minute, not less than 800 nl/minute, not less than 900 nl/minute, not less than 1 $\mu$l/minute, not less than 10 $\mu$l/minute, not less than 20 $\mu$l/minute, not less than 25 $\mu$l/minute, not less than 30 $\mu$l/minute, not less than 40 $\mu$l/minute, not less than 45 $\mu$l/minute, and not less than 50 $\mu$l/minute. The flow rate is preferably not less than 50 nl/minute. The flow rate is more preferably not less than 100 nl/minute. The flow rate is still more preferably not less than 120 nl/minute. The flow rate is still more preferably not less than 150 nl/minute. Further, the flow rate is still more preferably not less than 300 nl/minute, or not less than 450 nl/minute.

The laminar flow produces shear stress on the cell surface. Examples of the shear stress produced at the flow rate of the laminar flow in the one aspect of the present invention include, but are not limited to, not more than $8\times10^{-3}$ Pa, not more than $5\times10^{-3}$ Pa, not more than $4\times10^{-3}$ Pa, not more than $3\times10^{-3}$ Pa, not more than $2\times10^{-3}$ Pa, not more than $1\times10^{-3}$ Pa, not more than $9\times10^{-4}$ Pa, not more than $8.5\times10^{-4}$ Pa, not more than $1\times10^{-4}$ Pa, not more than $1\times10^{-5}$ Pa, not more than $1\times10^{-6}$ Pa, not more than $1\times10^{-7}$ Pa, and not more than $1\times10^{-8}$ Pa. The shear stress is preferably not more than $8\times10^{-3}$ Pa. The flow rate more preferably produces a microshear stress of not more than $5\times10^{-3}$ Pa, more preferably not more than $4\times10^{-3}$ Pa. The shear stress is still more preferably not more than $3\times10^{-3}$ Pa. Further, the shear stress is still more preferably not more than $2\times10^{-3}$ Pa, not more than $1\times10^{-3}$ Pa, not more than $9\times10^{-4}$ Pa, or not more than $8.5\times10^{-4}$ Pa. Further, examples of the shear stress produced at the flow rate of laminar flow include, but are not limited to, not less than $1\times10^{-8}$ Pa, not less than $1\times10^{-7}$ Pa, not less than $1\times10^{-6}$ Pa, not less than $1\times10^{-5}$

Pa, not less than $8\times10^{-5}$ Pa, not less than $1\times10^{-4}$ Pa, not less than $1.5\times10^{-4}$ Pa, not less than $2\times10^{-4}$ Pa, not less than $2.4\times10^{-4}$ Pa, not less than $4\times10^{-4}$ Pa, not less than $4.5\times10^{-4}$ Pa, not less than $7\times10^{-4}$ Pa, or not less than $7.5\times10^{-4}$ Pa. The shear stress is preferably not less than $8\times10^{-5}$ Pa. The shear stress is more preferably not less than $1.5\times10^{-4}$ Pa. The shear stress is still more preferably not less than $2\times10^{-4}$ Pa, and still more preferably not less than $2.4\times10^{-4}$ Pa. Further, the shear stress is still more preferably not less than $4\times10^{-4}$ Pa, not less than $4.5\times10^{-4}$ Pa, or not less than $7.5\times10^{-4}$ Pa.

In the one aspect of the present invention, the medium used for maintaining pluripotency may be any maintenance medium for embryonic stem cells that are commonly used for maintaining pluripotency of embryonic stem cells. Such a medium can be prepared using, as a basal medium, a medium used for culturing animal cells, and adding a desired substance(s) thereto. Examples of the basal medium include IMDM medium, Medium 199, Eagle's Minimum Essential Medium (EMEM), $\alpha$MEM, Dulbecco's modified Eagle's Medium (DMEM), Ham's F12 medium, RPMI 1640 medium and Fischer's medium, and mixtures of these media. For example, the medium may contain one or more substances selected from serum, albumin, transferrin, Knockout Serum Replacement (KSR) (serum replacement for FBS in ES cell culture), fatty acids, insulin, collagen precursor, trace elements, 2-mercaptoethanol, 3'-thiolglycerol, lipids, amino acids, L-glutamine, Glutamax (Invitrogen), non-essential amino acids, vitamins, bFGF, LIF, antibiotics, antioxidants, pyruvic acid, buffers and inorganic salts. The maintenance medium for embryonic stem cells may be a conditioned medium containing a culture supernatant of feeder cells such as fibroblasts or STO cells.

In the one aspect of the present invention, the culture of dispersed single cells can be carried out using a cell culture vessel combined with a liquid transferring device that can produce a laminar flow in the medium. The culture vessel is preferably coated, and examples of the coating agent include Matrigel (BD), collagen, gelatin, laminin, heparan sulfate proteoglycan and entactin, and combinations of these. The coating agent is preferably Matrigel.

The device that can produce a laminar flow in the medium may be an infusion pump that is well known to those skilled in the art, and examples of the device include peristaltic infusion pumps.

The size and shape of the cell culture vessel is not limited as long as the cell culture vessel has a size that allows culture of dispersed single pluripotent cells, and, in view of stably maintaining the laminar flow, the cell culture vessel may be one in which a medium is sent into a closed channel for culturing the cells. At this time, the cross-sectional shape of the channel is not limited, and may be any of polygons such as triangles, tetragons and pentagons; and circles. The shape is preferably a tetragon in view of simplicity of molding. The size of the cross-section of the channel is preferably smaller than the size of a circle having a diameter of 1 mm, and preferably larger than the size of a circle having a diameter of 0.1 mm. In the case of a polygon, each side may have a length of 1 nm to 500 $\mu$m. The cross-sectional area of the channel is, for example, within the range of 0.01 mm$^2$ to 1 mm$^2$. The area is preferably 0.25 mm$^2$. In the one aspect of the present invention, a channel whose cross-section is smaller than a circle having a diameter of 1 mm is referred to as a microchannel. The channel preferably has a tetragonal cross-section in which each side has a length of 500 $\mu$m. Examples of the shape of the channel include, but are not limited to, linear shapes, curved shapes and bent shapes. Further, the channel may be crossing another channel. The length of the channel is not limited, and may be not less than 1 mm. Examples of the length of the channel include 5 mm, 10 mm, 15 mm, 20 mm, 25 mm, 30 mm, 35 mm, 40mm, or lengths longer than these. The length is preferably 20 mm.

The cell culture vessel having a channel may be prepared with a material, for example, a plastic such as a silicone resin (e.g., polymethylsiloxane (PDMS)), polymethylmethacrylate, polyurethane, polystyrene or SU-8; or a glass. In this process, with a view to transferring a medium, to make the material hydrophilic, the inner wall portion of the channel may be subjected to oxygen plasma treatment of the material by a method well known to those skilled in the art. The channel of the cell culture vessel can be prepared by combining a layer having a groove to be used as the channel, with a flat layer. The groove may be molded using a mold prepared using a negative photoresist such as the SU-8 photoresist. For example, a photoresist is applied to a glass substrate, and the uncoated side is masked such that a desired shape as the channel is left unmasked. After performing exposure from the uncoated side and then removing the unexposed resist, a protrusion pattern remains on the glass substrate. This protrusion can be used as the mold for the groove. The height of the groove can be controlled by the application thickness of the photoresist.

In cases where a cell culture vessel having a channel is used, a solution reservoir for receiving the transferred medium, and an inlet for delivering cells and the like to the channel are preferably provided.

Further, pressurization is preferably carried out in order to avoid generation of air bubbles in the closed channel. The pressurization can be carried out by methods well known to those skilled in the art, such as increasing the liquid sending pressure generated by the pump, or compressing the channel. For example, in cases where a peristaltic infusion pump is used, the liquid sending pressure by the pump can be increased by increasing the tube thickness of the soft tube (about 400 $\mu$m) more than usual, and the soft tube is placed in the pump and used for transferring the liquid by squeezing. The method of compressing the channel may be, for example, attaching a diaphragm (e.g., a polyethylene membrane having a thickness of 30 $\mu$m) to the channel.

The above cell culture vessel and the liquid sending device may be placed, for example, at 37°C in the presence of 5% $CO_2$. Further, for maintenance culture, they may be placed under low-oxygen conditions. The term "low-oxygen conditions" herein means a state where the oxygen partial pressure is 1% to 10%, preferably 5%.

By allowing a medium, supplemented with a candidate agent as the subject of screening, to flow further in a cell culture vessel in which dispersed single pluripotent cells cultured by maintenance culture by the above method for culturing dispersed single cells are attached, and observing changes in the pluripotent cells, the effect of the candidate agent can be tested. Examples of the changes in the pluripotent cells herein include changes into specific cells such as endodermal cells, ectodermal cells, mesodermal cells, chorda-mesoderm, paraxial mesoderm, intermediate mesodermal cells, lateral plate mesodermal cells, nerve cells, glial cells, hematopoietic cells, hepatocytes, pancreatic beta cells, renal progenitor cells, endothelial cells, pericytes, epithelial cells, osteoblasts, myoblasts or chondrocytes. In such cases, the candidate agent may be selected as an inducer for differentiation into each type of cells. Examples of other modes include a test method for teratogenicity of a candidate agent, which method is carried out by observing changes in the growth of pluripotent cells using the method described in WO2010/035885. Accordingly, the one aspect of the present invention provides a cell culture vessel comprising dispersed single pluripotent cells, for screening of agents. For the purpose of decreasing the amount of the candidate agent as much as possible, the cell culture vessel preferably comprises a microchannel containing pluripotent cells.

EXAMPLES

The one aspect of the present invention is described below in more detail by way of the Examples below. However, the scope of the present invention is not limited by the Examples.

(Example 1) Preparation of Microfluidic Device

<Microfluidic Chip>

A polyethylene (PE) membrane 102, a polymethylsiloxane (PDMS) layer 103 and an SU8 layer 104, which is a glass plate to which SU8 was applied, were sandwiched between clamps 101 and 105, to construct a microfluidic chip 100 (Fig. 2). More specifically, the PDMS layer was prepared by pouring, to a thickness of 3 mm, a thermosetting PDMS (Slipot 184, Toray-Daw Corning, Japan) into a mold prepared by SU8 lithography for formation of 6 grooves (width, 0.5 mm; length, 20 mm; height, 0.5 mm), followed by curing at 80°C for 4 hours in a drier. By removing the cured PDMS from the mold, a PDMS layer with 6 channels formed thereon was obtained. At both ends of the channels in the PDMS layer, holes were made as inlets and outlets using a stainless steel biopsy needle having a diameter of 1 mm. The SU8 layer was prepared by applying SU8 (SU-8 2010, Microchem) to a glass plate and curing the SU8 by UV irradiation. A cross mark was given by SU8 lithography for adjustment of the XY axis during attachment to the PDMS layer. Subsequently, the surface of the SU8 layer was treated with oxygen plasma to add hydrophilicity thereto. The PE membrane was placed for reducing the pressure exerted by the clamps. In the clamp in the bottom side, a hole was formed such that the channel can be observed under the microscope. The layers were immobilized by sandwiching between the clamps.

<Construction of Microfluidic Device>

A peristaltic pump 5 composed of a tubing pump system with a silicone rubber tube having a tube thickness of 400 $\mu$m was connected to inlet 2 of the microfluidic chip 100 prepared by the above-described method via a tube (PTFE tube TUF-100 series, AWG-30, Chukoh Chemical Industries, Japan). Further, each inlet was connected to an injection valve 7 for placing a syringe for injecting cells. Further, outlet 3 was connected to a reservoir 6 (Fig. 3). To each of the inlet and outlet, a diaphragm 4 (a polyethylene membrane having a thickness of 30 $\mu$m) was attached to prevent generation of air bubbles in the channel. The thus prepared microfluidic device A (Fig. 1) could stably and accurately control the nanofluidic flow of the medium into the channel 1. The microfluidic device for a perfusion culture system was placed in a $CO_2$ incubator and operated with a DC power from a 3.0 V battery for several days.

(Example 2) Culture of iPS Cells Using Microfluidic Device, and Evaluation Thereof

<Preparation of iPS Cells>

OCT3/4, SOX2, KLF4 and c-MYC were introduced to human embryonic lung fibroblasts (TIG3) provided by the JCRB Cell Bank using a retrovirus, to induce an hiPSCs line. On a Matrigel-coated dish, TIG3-iPSC was cultured in an MEF (mouse embryonic fibroblast)-conditioned hES medium (DMEM/F12 supplemented with 20% knockout serum replacement (Invitrogen, Carlsbad, CA, USA), L-glutamine, non-essential amino acids, 2-mercaptoethanol and 10 ng/ml bFGF (Peprotech, Rocky Hill, NJ, USA)).

<Immunostaining>

The recovered cells were fixed using 4% PFA (paraformaldehyde)/PBS (phosphate buffered saline) at room temperature for 10 minutes, and washed in PBST (PBS supplemented with 0.1% Triton X-100), followed by performing pretreatment at 4°C overnight in a blocking solution (PBST supplemented with 3% BSA and 2% skim milk (DIFCO, USA)). Subsequently, immunoreaction was performed with primary antibody: anti-OCT4 (1:50, Santa Cruz Biotechnology, USA), anti-SOX2 (1:500, Abcam, Cambridge, UK) or anti-NANOG (1.200, Abcam, Cambridge, UK), and staining was then performed with a fluorescent secondary antibody (1:500, Invitrogen). Further, nuclei were counterstained with DAPI (4,6-diamidino-2-phenylindole), and photofading was prevented using SlowFade light antifade kit (Invitrogen), followed by obtaining fluorescent images using an IX70 inverted microscope.

<RT-PCR>

The cells were recovered, and total RNA was extracted from the cultured cells using TRIzol reagent (Invitrogen, USA). According to the manufacturer's instructions, cDNA was obtained from 1.0 $\mu$g of the total RNA using Superscript III (Invitrogen, USA) with random hexamers. Using, as a template, the thus obtained cDNA, and the primer sets described in Table 1, the genes of interest were amplified by PCR.

[Table 1]

| Gene | Forward (5'-3') | Reverse (5'-3') |
| --- | --- | --- |
| OCT4 | GCACTGTAGTGCTCGGTCCCTTTCCC | CTTCCCTCCAACCAGTTGCCCCAAAC |
| SOX2 | GGGAAATGGGAGGGGTGCAAAAGAGG | TTGCGTGAGTGTGGATGGGATTGGTG |
| NANOG | CAGCCCTGATTCTTCCACCAGTCCC | TGGAAGGTTCCCAGTCGGGTTCACC |
| GDF3 | CTTATGCTACGTAAAGGAGCGGG | GTGCCAACCCAG GTCCCGGAAGTT |
| REX1 | CAGATCCTAAACAGCTCGCAGAAT | GCGTACGCAAATTAAAGTCCAGA |
| NR0B1 | GTGGGGAACTCAGCAAATAC | GCATCATATCATCCATGCTG |
| STELLA | GCCTAGTGTTGTGTCAAGAG | GGTGCAAGAATAAGATTTATGGC |
| GAPDH | CTTCTTTTGGGTCGCCAGCCGAG | CAGCCTTGACGGTGCCATGGAA |

<Cell Culture Using Microfluidic Device>

The TIG3-iPSCs prepared by the above method were detached with 0.25% trypsin (Gibco)/0.04% EDTA, and recovered. The cells were then washed twice, and suspended in 500 $\mu$l of a fresh medium at $1.0 \times 10^5$ cells/ml. Using a syringe, Matrigel (BD) was allowed to flow from the injection valve into the channel, to introduce the cells in a singly dispersed state to the coated channel. The cells were cultured in a $CO_2$ incubator by perfusion with the above-described conditioned medium at a flow rate of 0 nl/minute or 500 nl/minute. The flow rate of 500 nl/minute is almost the same as the flow rate of the cilia-driven flow in the early mouse embryo that is required for causing left-right asymmetry. The cells on Day 1, Day 3 and Day 5 after the beginning of the perfusion culture were observed under a phase-contrast microscope (Fig. 4a). As a result, on Day 3 and Day 5 of the culture, it was found that, while the hiPSCs were dead under the static conditions, the hiPSCs cultured under the nanofluidic flow conditions survived to form colonies. The survival efficiencies of hiPSCs on Day 3 and Day 5 were significantly higher under the nanofluidic flow conditions than under the static conditions (Fig. 4b).

After 5 days of perfusion culture, normal culture was carried out on a Matrigel-coated dish, and the recovered cells were subjected to immunostaining as described above. As a result, expression of pluripotency marker proteins OCT4, NANOG and SOX2 could be confirmed (Fig. 4c). Similarly, as a result of RT-PCR carried out as described above on the recovered cells, expression of pluripotency marker genes OCT4, SOX2, NANOG, GDF3, REX1, DNMT3B and STELLA could be confirmed at levels equivalent to those observed during normal maintenance culture (Fig. 4d). Thus, it could be confirmed that perfusion culture under the nanofluidic flow allows culture of dispersed single hiPSCs while maintaining their pluripotency.

(Example 3) Evaluation of Flow Field Based on Simulation and Hydromechanics

<Confocal Microscope Analysis>

By confocal microscope analysis, hiPSCs on the channel were confirmed to have a volume of 550 $\mu m^3$, diameter of 24.5 $\mu m$ and height of 2.2 $\mu m$ (Fig. 5a). The confocal microscope used had the following constitution: microscope, IX71 (Olympus); confocal scan unit, CSU-X1 (Yokogawa Electric Corporation); and objective lens, 100× UPlanSAPO NA1.4 (Olympus).

<Calculation of Shear Stress Caused by Laminar Flow>

Using numeric codes obtained by the finite volume method (FVM) and the immersed boundary method, simulation based on numerical fluid dynamics was carried out. In terms of FVM, the continuity equation and Navier-Stokes equation were used after discretization. These were solved for the time-dependent, incompressible and three-dimensional conditions as described in Equations (1) and (2).
[Formula 1]

$$\frac{\partial U_i}{\partial x_i} = 0 \qquad (i=1, 2, 3) \qquad (1)$$

$$\rho_f \frac{DU_i}{Dt} = -\frac{\partial P}{\partial x_i} + \frac{\partial}{\partial x_j}\left(\mu \frac{\partial U_i}{\partial x_j}\right) \qquad (2)$$

As the fluid and fin, properties of water and stainless steel were used, respectively. For discretization of Equation (2), the second-order accurate central difference was used as the diffusion term, and the first-order accurate upwind difference was used as the convection term. For pressure correction, the SIMPLE (Semi-implicit method for pressure-linked equations) algorithm was used. By linear iteration using TDMA (Tri-Diagonal Matrix Algorithm) combined with ADI (Alternating Direction Implicit), the discretized algebraic equation was solved. As a result, the shear stress on the surface of a single hiPSC in a nanofluidic flow of a medium at 500 nl/minute in a channel (height = 500 $\mu m$ × width = 500 $\mu m$) was roughly estimated to be not more than about $1.0 \times 10^{-3}$ Pa (Fig. 5b). This was calculated based on the fact that the flow rate of the nanofluidic flow became about 0.5 $\mu m$/second at a distance of about 250 nm from the surface of hiPSCs (Fig. 5c). A similar flow rate is reported to be sufficient for inducing a molecular response in a specific type of mammalian cells due to a change in expression of a gene (Wimmer MA, et al. J Biomech. 2009, 42:424-429).

(Example 4) Study of Flow Rate in iPS Cell Culture Using Microfluidic Device

In order to study the optimum flow rate, a study was carried out on culture of dispersed single hiPS cells in a nanofluidic flow at $5 \times 10^2$ nl/minute or $5 \times 10^4$ nl/minute. As a result, with a shear stress of 0.1 Pa caused by the nanofluidic flow at $5 \times 10^4$ nl/minute, no attached hiPSCs were detected (Fig. 5d).
In order to further study the optimum flow rate, a cell suspension prepared by dispersing single cells in 0.25% trypsin/EDTA at $10^5$ cells/ml was injected into a Matrigel-coated channel, and, after confirming their adhesion, the cells were perfused with the MEF-conditioned hES medium at 37°C under an atmosphere of 5% $CO_2$ at various flow rates (0, 150, 300, 500, 1000 and 2000 nl/minute; which correspond to shear stresses of 0, $2.4 \times 10^{-4}$, $4.8 \times 10^{-4}$, $8.0 \times 10^{-4}$, $1.6 \times 10^{-3}$, and $3.2 \times 10^{-3}$ Pa). Matrigel coating of the inside of the channel was carried out by perfusion with Matrigel diluted with DMEM, at 37°C under an atmosphere of 5% $CO_2$ for a day and a night. Three days after the perfusion, the cells in the channel were detached with 0.25% trypsin/EDTA and then recovered, followed by counting the number of Nanog-positive living cells. The ratio of the number of cells on Day 3 after the perfusion to the number of cells counted on Day 1 after the perfusion in the same manner was calculated as the survival rate. As a result, it was found that, at a flow rate of $5 \times 10^2$ nl/minute, the survival rate was not less than 100%, and therefore that survival of the dispersed single iPS cells was possible (Fig. 6).
An attempt was made to compare the chance (frequency) of cell-cell contact between a microchannel and a culture dish based on the area occupied by each cell. As a result, there was no large difference in the intercellular distance between these since the density was 35 cells/mm$^2$ in the microchannel (based on the assumption that the number of cells in the channel is 100 to 350 and the base area of each channel is 10 mm$^2$ (calculated with 20×0.5 mm)) and 176 cells/mm$^2$ in the culture dish (based on the assumption that 5 ml of a cell suspension (10$^5$ cells/ml) is placed in a 6-cm Petri dish). Therefore, the survival of iPS cells that were dispersed at the single-cell level observed in the microchannel is assumed to be due to influences of: (1) the flow of the culture liquid in the vicinity; and (2) the microculture environment; which are characteristic to microchannels. Further, since an increased survival rate was observed at a flow rate of 500 nl/min., it was found in the present Example that the survival of iPS cells was influenced by both the flow in their vicinity and the microculture environment.

INDUSTRIAL APPLICABILITY

In the culture method of the one aspect of the present invention, pluripotent cells are cultured in a state where the cells are dispersed as single cells, to enable production of their cloned cells. Therefore, the one aspect of the present invention can be used for quality control of iPSCs for application to regenerative medicine, improvement of the efficiency of gene manipulation, improvement of the efficiency of cloning, and the like.

## Claims

1. A method for culturing dispersed single pluripotent cells wherein said dispersed cells are separated cells that do not show joining of two or more cells via cadherin, said method comprising culturing adhered dispersed single cells under laminar flow conditions without addition of an anti-apoptotic agent.

2. The method according to claim 1, wherein said cells are cultured on Matrigel.

3. The method according to claim 1 or 2, wherein an embryonic stem cell-maintenance medium is allowed to flow as a laminar flow.

4. The method according to claim 3, wherein said embryonic stem cell-maintenance medium comprises a culture supernatant of fibroblasts.

5. The method according to any one of claims 1 to 4, wherein said cells are cultured in a cylindrical container whose cross-section is a circle having a diameter of not more than 1 mm.

6. The method according to claim 5, wherein said cells are cultured in a container having a width of 0.1 mm to 1 mm, height of 0.1 mm to 1 mm and length of 5 to 40 mm.

7. The method according to any one of claims 1 to 6, wherein said laminar flow has a flow rate of not more than 50 $\mu$l/min.

8. The method according to claim 7, wherein said laminar flow has a flow rate of 1 nl/min. to 50 $\mu$l/min.

9. The method according to claim 7, wherein said laminar flow has a flow rate of 50 nl/min. to 5 $\mu$l/min.

10. The method according to any one of claims 1 to 9, wherein said laminar flow has a flow rate that causes a microshear stress of not more than $8 \times 10^{-3}$ Pa on the cell surface.

11. The method according to any one of claims 1 to 10, wherein said laminar flow has a flow rate that causes a microshear stress of not less than $8 \times 10^{-5}$ Pa on the cell surface.

12. The method according to any one of claims 1 to 11, wherein said pluripotent cells are human pluripotent cells.

13. The method according to claim 12, wherein said pluripotent cells are embryonic stem cells or induced pluripotent stem cells.

14. The method according to any one of claims 1 to 13, wherein said laminar flow is produced with a peristaltic pump.

**Patentansprüche**

1. Verfahren zum Züchten dispergierter einzelner pluripotenter Zellen, wobei die dispergierten Zellen getrennte Zellen sind, die keine Verbindung von zwei oder mehreren Zellen durch Cadherin aufweisen, wobei das Verfahren das Züchten haftender dispergierter einzelner Zellen unter laminaren Strömungsbedingungen ohne Zugabe eines anti-apoptotischen Mittels umfasst.

2. Verfahren nach Anspruch 1, wobei die Zellen auf Matrigel gezüchtet werden.

3. Verfahren nach Anspruch 1 oder 2, wobei einem Embryonalstammzellen-Maintenancemedium gestattet wird, als laminare Strömung zu strömen.

4. Verfahren nach Anspruch 3, wobei das Embryonalstammzellen-Maintenancemedium einen Züchtungsüberstand aus Fibroblasten umfasst.

5. Verfahren nach irgendeinem der Ansprüche 1 bis 4, wobei die Zellen in einem zylindrischen Behälter gezüchtet werden, dessen Querschnitt ein Kreis mit einem Durchmesser von nicht mehr als 1 mm ist.

6. Verfahren nach Anspruch 5, wobei die Zellen in einem Behälter gezüchtet werden, der eine Breite von 0,1 mm bis 1 mm, eine Höhe von 0,1 mm bis 1 mm und eine Länge von 5 bis 40 mm aufweist.

7. Verfahren nach einem der Ansprüche 1 bis 6, wobei die laminare Strömung eine Strömungsrate von nicht mehr als 50 $\mu$l/min aufweist.

8. Verfahren nach Anspruch 7, wobei die laminare Strömung eine Strömungsrate von 1 nl/min bis 50 $\mu$l/min aufweist.

9. Verfahren nach Anspruch 7, wobei die laminare Strömung eine Strömungsrate von 50 nl/min bis 5 $\mu$l/min aufweist.

10. Verfahren nach irgendeinem der Ansprüche 1 bis 9, wobei die laminare Strömung eine Strömungsrate aufweist, die eine Microscherbeanspruchung von nicht mehr als $8 \times 10^{-3}$ Pa auf der Zelloberfläche verursacht.

11. Verfahren nach irgendeinem der Ansprüche 1 bis 10, wobei die laminare Strömung eine Strömungsrate aufweist, die eine Microscherbeanspruchung von nicht mehr als $8 \times 10^{-5}$ Pa auf der Zelloberfläche verursacht.

12. Verfahren nach irgendeinem der Ansprüche 1 bis 11, wobei die pluripotenten Zellen humane pluripotente Zellen sind.

13. Verfahren nach Anspruch 12, wobei die pluripotenten Zellen Embryonalstammzellen oder induzierte pluripotente Stammzellen sind.

**14.** Verfahren nach irgendeinem der Ansprüche 1 bis 13, wobei die laminare Strömung mit einer peristaltischen Pumpe erzeugt wird.

**Revendications**

**1.** Procédé de culture de cellules pluripotentes unitaires dispersées, dans lequel lesdites cellules dispersées sont des cellules séparées qui ne montrent pas de liaison par la cadhérine entre deux cellules ou plus, ledit procédé comprenant une culture de cellules unitaires dispersées adhérées sous des conditions de flux laminaire sans un ajout d'un agent anti-apoptotique.

**2.** Procédé selon la revendication 1, dans lequel lesdites cellules sont cultivées sur Matrigel.

**3.** Procédé selon la revendication 1 ou 2, dans lequel un milieu de conservation de cellules souches embryonnaires peut s'écouler en tant que flux laminaire.

**4.** Procédé selon la revendication 3, dans lequel ledit milieu de conservation de cellules souches embryonnaires comprend un surnageant de culture de fibroblastes.

**5.** Procédé selon l'une quelconque des revendications 1 à 4, dans lequel lesdites cellules sont cultivées dans un récipient cylindrique dont la section transversale est un cercle ayant un diamètre d'au plus 1 mm.

**6.** Procédé selon la revendication 5, dans lequel lesdites cellules sont cultivées dans un récipient ayant une largeur de 0,1 mm à 1 mm, une hauteur de 0,1 mm à 1 mm et une longueur de 5 à 40 mm.

**7.** Procédé selon l'une quelconque des revendications 1 à 6, dans lequel ledit flux laminaire a un débit d'au plus 50 $\mu$l/min.

**8.** Procédé selon la revendication 7, dans lequel ledit flux laminaire a un débit de 1 nl/min à 50 $\mu$l/min.

**9.** Procédé selon la revendication 7, dans lequel ledit flux laminaire a un débit de 50 nl/min à 5 $\mu$l/min.

**10.** Procédé selon l'une quelconque des revendications 1 à 9, dans lequel ledit flux laminaire a un débit qui entraîne une micro-contrainte de cisaillement d'au plus $8 \times 10^{-3}$ Pa sur la surface de la cellule.

**11.** Procédé selon l'une quelconque des revendications 1 à 10, dans lequel ledit flux laminaire a un débit qui entraîne une micro-contrainte de cisaillement d'au moins $8 \times 10^{-5}$ Pa sur la surface de la cellule.

**12.** Procédé selon l'une quelconque des revendications 1 à 11, dans lequel lesdites cellules pluripotentes sont des cellules pluripotentes humaines.

**13.** Procédé selon la revendication 12, dans lequel lesdites cellules pluripotentes sont des cellules souches embryonnaires ou des cellules souches pluripotentes induites.

**14.** Procédé selon l'une quelconque des revendications 1 à 13, dans lequel ledit flux laminaire est produit par une pompe péristaltique.

[Fig. 1]

[Fig. 2]

[Fig. 3]

(a)

100

1

2   4   3

(b)

2   4   3   102
103
104
105
1

5

(c)

6   2   1   3

[Fig. 4]

[Fig. 5]

a

diameter = Φ 24.5 ± 4.7 x H 2.2 ± 0.3 μm (n = 13)

b

c

d

Flow rate (nl/min)

5 x 10²     5 x 10⁴

[Fig. 6]

## REFERENCES CITED IN THE DESCRIPTION

**Patent documents cited in the description**

- US 5843780 A
- WO 2007069666 A
- WO 2008118820 A
- WO 2009007852 A
- WO 2009032194 A
- WO 2009058413 A
- WO 2009057831 A
- WO 2009075119 A
- WO 2009079007 A
- WO 2009091659 A
- WO 2009101084 A
- WO 2009101407 A
- WO 2009102983 A
- WO 2009114949 A
- WO 2009117439 A
- WO 2009126250 A
- WO 2009126251 A
- WO 2009126655 A
- WO 2009157593 A
- WO 2010009015 A
- WO 2010033906 A
- WO 2010033920 A
- WO 2010042800 A
- WO 2010050626 A
- WO 2010056831 A
- WO 2010068955 A
- WO 2010098419 A
- WO 2010102267 A
- WO 2010111409 A
- WO 2010111422 A
- WO 2010115050 A
- WO 2010124290 A
- WO 2010147395 A
- WO 2010147612 A
- WO 2010008054 A
- WO 2010137746 A
- WO 2009123349 A
- WO 2010013845 A
- WO 2011007900 A
- WO 2010035885 A
- EP 12842151 A
- JP 2011232097 A

**Non-patent literature cited in the description**

- **THOMSON, J. A. et al.** *Science,* 1998, vol. 282, 1145-1147
- **TAKAHASHI K ; YAMANAKA S.** *Cell,* 2006, vol. 126, 663-676
- **OHGUSHI M.** *Cell Stem Cell,* 2010, vol. 7, 225-239
- **BURDINE RD ; SCHIER AF.** *Genes Dev.,* 2000, vol. 14, 763-776
- **TOH YC ; VOLDMAN J.** *FASEB J.,* vol. 201 (25), 1208-1217
- **TESAR PJ.** *Nature,* 2007, vol. 448, 196-199
- **M. J. EVANS ; M. H. KAUFMAN.** *Nature,* 1981, vol. 292, 154-156
- **J. A. THOMSON et al.** *Science,* 1998, vol. 282, 1145-1147
- **J. A. THOMSON et al.** *Proc. Natl. Acad. Sci. USA,* 1995, vol. 92, 7844-7848
- **J. A. THOMSON et al.** *Biol. Reprod.,* 1996, vol. 55, 254-259
- **J. A. THOMSON ; V. S. MARSHALL.** *Curr. Top. Dev. Biol.,* 1998, vol. 38, 133-165
- **THOMSON JA et al.** *Proc Natl. Acad. Sci. U S A.,* 1995, vol. 92, 7844-7848
- **THOMSON JA et al.** *Science,* 1998, vol. 282, 1145-1147
- **H. SUEMORI et al.** *Biochem. Biophys. Res. Commun.,* 2006, vol. 345, 926-932
- **M. UENO et al.** *Proc. Natl. Acad. Sci. USA,* 2006, vol. 103, 9554-9559
- **H. SUEMORI et al.** *Dev. Dyn.,* 2001, vol. 222, 273-279
- **H. KAWASAKI et al.** *Proc. Natl. Acad. Sci. USA,* 2002, vol. 99, 1580-1585
- **KLIMANSKAYA I et al.** *Nature,* 2006, vol. 444, 481-485
- **O. FUMITAKA et al.** *Nat. Biotechnol.,* 2008, vol. 26, 215-224
- **E. KROON et al.** *Nat. Biotechnol.,* 2008, vol. 26, 443-452
- **M. KANATSU-SHINOHARA et al.** *Biol. Reprod.,* 2003, vol. 69, 612-616
- **K. SHINOHARA et al.** *Cell,* 2004, vol. 119, 1001-1012
- **MASANORI TAKEHASHI et al.** Experimental Medicine. Yodosha, 2008, vol. 26, 41-46
- **Y. MATSUI et al.** *Cell,* 1992, vol. 70, 841-847

- **J. L. RESNICK et al.** *Nature,* 1992, vol. 359, 550-551
- **K. TAKAHASHI ; S. YAMANAKA.** *Cell,* 2006, vol. 126, 663-676
- **K. TAKAHASHI et al.** *Cell,* 2007, vol. 131, 861-872
- **J. YU et al.** *Science,* 2007, vol. 318, 1917-1920
- **NAKAGAWA, M. et al.** *Nat. Biotechnol.,* 2008, vol. 26, 101-106
- **HUANGFU D et al.** *Nat. Biotechnol.,* 2008, vol. 26, 795-797
- **SHI Y et al.** *Cell Stem Cell,* 2008, vol. 2, 525-528
- **EMINLI S et al.** *Stem Cells,* 2008, vol. 26, 2467-2474
- **HUANGFU D et al.** *Nat Biotechnol.,* 2008, vol. 26, 1269-1275
- **SHI Y et al.** *Cell Stem Cell,* 2008, vol. 3, 568-574
- **ZHAO Y et al.** *Cell Stem Cell,* 2008, vol. 3, 475-479
- **MARSON A.** *Cell Stem Cell,* 2008, vol. 3, 132-135
- **FENG B et al.** *Nat Cell Biol.,* 2009, vol. 11, 197-203
- **R.L. JUDSON et al.** *Nat. Biotech.,* 2009, vol. 27, 459-461
- **LYSSIOTIS CA et al.** *Proc Natl Acad Sci U S A.,* 2009, vol. 106, 8912-8917
- **KIM JB et al.** *Nature,* 2009, vol. 461, 649-643
- **ICHIDA JK et al.** *Cell Stem Cell.,* 2009, vol. 5, 491-503
- **HENG JC et al.** *Cell Stem Cell,* 2010, vol. 6, 167-74
- **HAN J et al.** *Nature,* 2010, vol. 463, 1096-100
- **MALI P et al.** *Stem Cells,* 2010, vol. 28, 713-720
- **MAEKAWA M et al.** *Nature,* 2011, vol. 474, 225-9
- *Cell,* 2006, vol. 126, 663-676
- *Cell,* 2007, vol. 131, 861-872
- *Science,* 2007, vol. 318, 1917-1920
- *Science,* 2008, vol. 322, 945-949
- *Science,* 2008, vol. 322, 949-953
- **WARREN L.** *Cell Stem Cell,* 2010, vol. 7, 618-630
- **TAKAHASHI K et al.** *PLoS One,* 2009, vol. 4, e8067
- **SUN N et al.** *Proc Natl Acad Sci U S A.,* 2009, vol. 106, 15720-15725
- **YOSHIDA Y et al.** *Cell Stem Cell,* 2009, vol. 5, 237-241
- **WIMMER MA et al.** *J Biomech.,* 2009, vol. 42, 424-429